(19) Europäisches Patentamt / European Patent Office / Office européen des brevets

(11) **EP 2 277 871 A1**

(12) **EUROPEAN PATENT APPLICATION**
published in accordance with Art. 153(4) EPC

(43) Date of publication:
**26.01.2011 Bulletin 2011/04**

(21) Application number: **09738887.0**

(22) Date of filing: **01.05.2009**

(51) Int Cl.:
***C07D 307/28*** *(2006.01)* ***C07B 61/00*** *(2006.01)*
***C07C 41/32*** *(2006.01)* ***C07C 43/15*** *(2006.01)*

(86) International application number:
**PCT/JP2009/058570**

(87) International publication number:
**WO 2009/133950 (05.11.2009 Gazette 2009/45)**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: **01.05.2008 JP 2008119969**

(71) Applicant: **Kuraray Co., Ltd.**
**Okayama 710-8622 (JP)**

(72) Inventor: **FUJI, Junichi**
**Kamisu-shi**
**Ibaraki 314-0197 (JP)**

(74) Representative: **Vossius & Partner**
**Siebertstrasse 4**
**81675 München (DE)**

(54) **METHOD FOR PRODUCING VINYL ETHER COMPOUND**

(57) The invention provides a method for producing a vinyl ether compound, **characterized in that** the method includes isomerizing an allyl ether compound represented by formula (I) or (II):

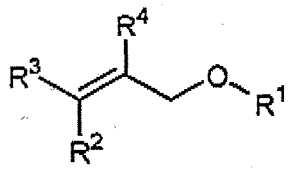

(I)

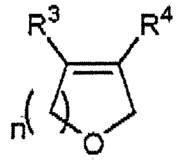

(II)

(wherein $R^1$ represents a C1 to C6 alkyl group; each of $R^2$, $R^3$, and $R^4$ independently represents a hydrogen atom, a C1 to C6 alkyl group, or a C3 to C6 alkenyl group; and n is 1 or 2) in the presence of hydrogen, a monodentate tris(ortho-substituted aryl) phosphite, and a rhodium compound. The vinyl ether compound is useful as a raw material, an intermediate, etc. for producing pharmaceuticals, agrochemicals, polymers, etc.

EP 2 277 871 A1

## Description

Technical Field

The present invention relates to a method for producing a vinyl ether compound and, more particularly, to a method for producing, in an industrially advantageous manner, a vinyl ether compound such as 2,3-dihydrofuran, which method employs a monodentate tris(ortho-substituted aryl) phosphite and a rhodium compound in the presence of hydrogen.

Background Art

Hitherto, there have been known methods for producing a vinyl ether compound comprising isomerization of an allyl ether compound, such as a method which employs a polyoxyalkylene-chain-containing compound in the presence of an alkali metal hydroxide and/or an alkaline earth metal hydroxide as a catalyst (see Patent Document 1); a method for continuously producing dihydrofuran comprising isomerization of 2,5-dihydrofuran in the presence of a palladium catalyst and a platinum-on-carrier catalyst (see Patent Document 2); a method comprising heating 2,5-dihydrofuran at 20 to 100°C in the presence of a tertiary phosphine and, as a homogeneous noble metal catalyst, a ruthenium catalyst or a rhodium catalyst (see Patent Document 3); and a method comprising isomerization of an allyl ether compound to a corresponding enol ether compound in the presence of a ruthenium catalyst or a rhodium catalyst and triphenylphosphine under argon or $CO/H_2$ mixture (see Patent Document 4).
Patent Document 1: Japanese Patent Application Laid-Open (*kokai*) No. Hei 9-157201
Patent Document 2: Japanese *Kohyo* (PCT) Patent Publication No. 2000-510118
Patent Document 3: US Patent No. 5,254,701
Patent Document 4: Japanese *Kohyo* (PCT) Patent Publication No. Hei 9-508109, Examples 10 and 11

Disclosure of the Invention

Problems to be Solved by the Invention

When the method disclosed in Patent Document 1 is employed, the used alkaline substance must be removed by use of water, possibly causing loss of the target compound into an aqueous phase. In addition, the wash water must be neutralized with acid before discharge thereof, making the method cumbersome and possibly imposing a heavy load to the environment.
In the method disclosed in Patent Document 2, employing a palladium catalyst and a platinum-on-carrier catalyst, the catalyst can be readily removed from the reaction system. In addition, disproportionation of 2,3-dihydrofuran can be prevented through controlling the percent conversion at one batch of reaction to 40 to 60%, whereby a high production yield of 97% or higher is attained. However, the difference in boiling point between the raw material and the product of interest is so small that effective separation of the two species is difficult. Furthermore, this method requires a distillation tower having 20 or more plates, imposing high cost on the facility. Due to such a small difference in boiling point, utilities unavoidably increase.
The method disclosed in Patent Document 3 have attained a 2,3-dihydrofuran yield of ≥98%. However, when the present inventor previously performed the reaction in accordance with Example 4 of Patent Document 3 (see Comparative Example 2 of the present specification), the reaction problematically stopped in an incomplete state with a low production yield in the case where 2,5-dihydrofuran serving as a raw material contained a trace amount of impurities, failing to consistently produce 2,3-dihydrofuran. Thus, this method must be further improved.
The method disclosed in Patent Document 4 has attained a percent selectivity of ≥90%, as is clear from Examples 10 and 11. However, the percent conversion remained about 50%. In fact, when the present inventor previously performed the reaction in accordance with Example 11 of Patent Document 4, percent conversion was considerably low (see Comparative Example 6 of the present specification). Patent Document 4 discloses that the catalyst concentration in the isomerization step is 0.05 to 0.2 mol% with respect to the allyl ether compound serving as the raw material (see p. 34, lines 1 to 4 from the bottom). Thus, the amount of the transition noble metal catalyst employed is considerably large, and such an expensive transition noble metal catalyst cannot be used. When the present inventor conducted a similar experiment under the conditions disclosed in Patent Document 4, with changing the raw material to 2,5-dihydrofuran, difficulty was encountered in repeated use of the catalyst 5 times or more. Thus, this method must be further improved for industrially carrying out the method in an advantageous manner.

Means for Solving the Problems

In order to attain the aforementioned objects, the present inventor has conducted extensive studies, and has found that through isomerization of an allyl ether compound by use in combination of a rhodium compound and a monodentate tris(ortho-substituted aryl) phosphite as a catalyst in the presence of hydrogen, [1] a vinyl ether compound can be produced by use of a just small amount of a rhodium compound, with high percent conversion of an allyl ether compound and high percent selectivity to the vinyl ether compound; [2] a vinyl ether compound can be produced at high yield without being affected by impurities, even when the starting allyl ether compound contains impurities; and [3] the aforementioned catalyst can be used repeatedly, and formation of high-boiling-point fractions is suppressed in isomerization in the presence of a tertiary amine, ensuring the merit of repeated use of the catalyst. The present invention has been accomplished on the basis of this finding.

Accordingly, the present invention provides the following (1) to (6). (1) A method for producing a vinyl ether compound, **characterized in that** the method comprises isomerizing an allyl ether compound represented by formula (I) or (II) :

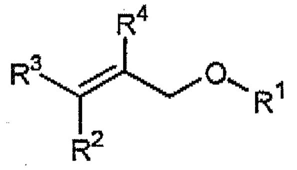

(I)

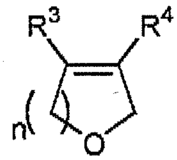

(II)

(wherein $R^1$ represents a C1 to C6 alkyl group; each of $R^2$, $R^3$, and $R^4$ independently represents a hydrogen atom, a C1 to C6 alkyl group, or a C3 to C6 alkenyl group; and n is 1 or 2) in the presence of hydrogen, a monodentate tris(ortho-substituted aryl) phosphite, and a rhodium compound.

(2) A method for producing a vinyl ether compound as described in (1) above, wherein the monodentate tris(ortho-substituted aryl) phosphite mole concentration of the reaction mixture is adjusted to 0.9 to 15 times the sum of the peroxide mole concentration of the reaction mixture and the rhodium atom mole concentration of the reaction mixture, the peroxide being originally contained in the raw material, and isomerization is performed at a reaction temperature of 60 to 180°C in the absence of carbon monoxide after adjusting the hydrogen pressure to 0.1 to 0.9 MPa (absolute pressure).

(3) A method for producing a vinyl ether compound as described in (1) or (2) above, wherein the reaction mixture has a rhodium atom mole concentration of 0.01 to 2 mmol/L.

(4) A method for producing a vinyl ether compound as described in (1) or (2) above, wherein isomerization is performed further in the presence of a tertiary amine having a boiling point of 100°C or higher.

(5) A method for producing a vinyl ether compound as described in (1) or (2) above, wherein the monodentate tris (ortho-substituted aryl) phosphite is at least one species selected from among tris(2-t-butylphenyl) phosphite, tris (3-methyl-6-t-butylphenyl) phosphite, and tris(2,4-di-t-butylphenyl) phosphite.

(6) A method for producing a vinyl ether compound as described in (1) or (2) above, wherein the allyl ether compound serving as a raw material is 2,5-dihydrofuran containing, as impurities, furan in an amount of 0.1 to 7 mass%, water in an amount of 0.5 to 23 mass%, and crotonaldehyde in an amount of 0.01 to 5 mass%.

Effects of the Invention

According to the present invention, a vinyl ether compound can be produced from an allyl ether compound by use of a small amount of a rhodium compound, with high percent conversion and high percent selectivity.

Best Modes for Carrying out the Invention

In the present invention, an allyl ether compound represented by formula (I) or (II):

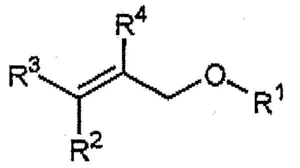

(I)

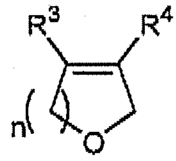

(II)

(hereinafter may be referred to simply as "allyl ether compound") is isomerized in the presence of hydrogen, a monodentate tris(ortho-substituted aryl) phosphite, and a rhodium compound.

In the aforementioned formulas (I) and (II), $R^1$ represents a $C^1$ to C6 alkyl group; each of $R^2$, $R^3$, and $R^4$ independently represents a hydrogen atom, a C1 to C6 alkyl group, or a C3 to C6 alkenyl group; and n is 1 or 2.

Examples of the C1 to C6 alkyl group of represented by each of $R^1$, $R^2$, $R^3$, and $R^4$ include a methyl group, an ethyl group, various form propyl groups (the various form groups means that the groups includ a linear-chain group and any branched-chain, and the same is applied to the entire specification), and various form butyl groups.

Examples of the C3 to C6 alkenyl group of represented by each of $R^2$, $R^3$, and $R^4$ include 2-propenyl group, 2-butenyl group, 3-butenyl group, 2-pentenyl group, 3-pentenyl group, 4-pentenyl group, 2-hexenyl group, 3-hexenyl group, 4-hexenyl group, and 5-hexenyl group. The alkenyl group is preferably an alkenyl group having no C-C double bond between the 1,2-positions, from the viewpoint of suppression of side reaction.

In the aforementioned formula (I), $R^1$ is preferably methyl group, ethyl group, or t-butyl group. $R^2$ is preferably a hydrogen atom. $R^3$ is preferably a hydrogen atom, a methyl group, an ethyl group, a n-propyl group, or a 4-pentenyl group. $R^4$ is preferably a hydrogen atom.

In the aforementioned formula (II), $R^3$ is preferably a hydrogen atom. $R^4$ is preferably a hydrogen atom or a methyl group. n is preferably 1.

Specific examples of the allyl ether compound include allyl ethyl ether, allyl t-butyl ether, 2-butenyl methyl ether, 2-pentenyl methyl ether, 2-hexenyl methyl ether, 2,7-octadienyl methyl ether, 2,5-dihydrofuran, 3,6-dihydro-2H-pyran, and 3,6-dihydro-4-methyl-2H-pyran.

When the allyl ether compound represented by formula (I) is employed as a raw material, the vinyl ether compound represented by the following formula (I') is a target compound. When formula the allyl ether compound represented by formula (II) is employed as a raw material, the vinyl ether compound represented by the following formula (II') is a target compound. In the formulas (I') and (II'), $R^1$ to $R^4$ have the same meanings as defined above.

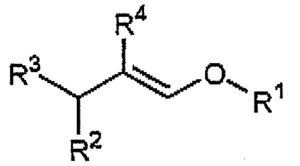

(I')

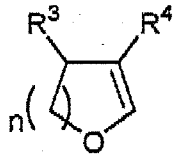

(II')

The monodentate tris(ortho-substituted aryl) phosphite employed in the present invention is a compound represented by the following formula (III):

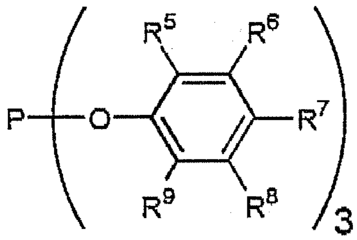

(III)

(wherein $R^5$ represents an alkyl group or an aryl group; each of $R^6$ to $R^9$ independently represents a hydrogen atom or an alkyl group).
The number of carbon atoms of the alkyl group $R^5$ is preferably 1 to 10, more preferably 1 to 6, still more preferably 3 to 6. Specific examples of the alkyl group $R^5$ include methyl group, ethyl group, various form propyl groups, various form butyl groups, various form hexyl groups, various form octyl groups, and various form decyl groups.
The number of carbon atoms of the aryl group $R^5$ is preferably 6 to 10. Examples of the aryl group $R^5$ include phenyl group and tolyl groups.
$R^5$ is preferably an alkyl group, more preferably a t-butyl group.
The number of carbon atoms of the alkyl group represented by each of $R^6$ to $R^9$ is preferably 1 to 10, more preferably 1 to 6. Specific examples of the alkyl group represented by each of $R^6$ to $R^9$ include methyl group, ethyl group, various form propyl groups, various form butyl groups, various form hexyl groups, various form octyl groups, and various form decyl groups.
Each of $R^6$ to $R^9$ is preferably a hydrogen atom, a methyl group, or a t-butyl group.

Specific examples of the monodentate tris(ortho-substituted aryl) phosphite include tris(2-t-butylphenyl)phasphite, tris(3-methyl-6-t-butylphenyl)phosphite, tris(2,4-di-t-butylphenyl)phosphite, and tris(2-phenylphenyl)phosphite. of these, from the viewpoints of percent conversion of the allyl ether compound and yield of and selectivity to the vinyl ether compound, the monodentate tris(ortho-substituted aryl) phosphite is preferably at least one species selected from the group consisting of tris(2-t-butylphenyl)phosphite, tris(3-methyl-6-t-butylphenyl)phosphite, and tris(2,4-di-t-butylphenyl) phosphite.
Through use, in combination, of such a monodentate tris(ortho-substibstituted aryl) phosphite and a rhodium compound as a combination catalyst, a vinyl ether compound of interest can be produced at high yield without being adversely affected, even though the allyl ether compound serving as a raw material contains impurities, and the rhodium compound is used in a small amount.

Examples of the rhodium compound employed in the present invention include rhodium complexes such as $Rh(acac)(CO)_2$, $Rh(acac)_3$, $[Rh(OAc)(CO)_2]_2$, $Rh(OAc)_3$, $Rh_2(OAc)_2(1,5\text{-}COD)_2$, $Rh(CO)(acac)(PPh_3)$, $HRh(CO)(PPh_3)_3$, and $Rh_4(CO)_{12}$ (wherein acas represents an acetylacetonato group, Ac represents an acetyl group, COD represents 1,5-cyclooctadiene, and Ph represents a phenyl group). Of these, $Rh(acac)(CO)_2$ is preferred. These rhodium complexes may be commercial products or synthesized products obtained through a known method.
These rhodium compounds may be used singly or in combination of two or more species.

Since the aforementioned rhodium compound is expensive, the amount of rhodium compound used with respect to the produced vinyl ether compound must be reduced to as low a level as possible.
The amount of the rhodium compound used in the present invention, as reduced to the rhodium atom mole concentration of the reaction mixture, may be adjusted to 0.01 to 2 mmol/L. If required, the amount may be adjusted to 0.1 to 1 mmol/L.

In one embodiment of the present invention, the monodentate tris(substituted aryl) phosphite mole concentration of the reaction mixture is adjusted to 0.9 to 15 times the sum of the peroxide mole concentration of the reaction mixture and the rhodium atom mole concentration of the reaction mixture, the peroxide being originally contained in the raw material, and isomerization is performed at a reaction temperature of 60 to 180°C after adjusting the hydrogen pressure to 0.1 to 0.9 MPa (absolute pressure). In this embodiment, higher percent conversion and percent selectivity can be attained by use of a small amount of rhodium compound. The conditions employed in the embodiment are preferred. In addition, the present invention is preferably carried out in the absence of carbon monoxide from the viewpoint of suppression of side reactions.
No particular limitation is imposed on the method of determining the substance mole concentration of the reaction mixture. In one procedure, the amount of monodentate tris(ortho-substituted aryl) phosphite or rhodium compound is determined with respect to the total volume (1 L) of the allyl ether compound fed to the reactor, reaction products, and solvent (in the specification, the mixture is referred to as "reaction mixture"). The peroxide mole concentration of the reaction mixture,

the peroxide being originally contained in the allyl ether compound, may be calculated through the method described in the Examples hereinbelow.

Generally, allyl ether compounds serving as a raw material of the present invention, in particular 2,5-dihydrofuran, readily form a peroxide. The monodentate tris(ortho-substituted aryl) phosphite used in the present invention tends to be oxidized by such a peroxide. Thus, in order to consistently perform reaction, the amount of monodentate tris(ortho-substituted aryl) phosphite used in the invention is preferably selected in consideration of the peroxide mole concentration of the reaction mixture, the peroxide originallt contained the raw material.

Therefore, as described above, the monodentate tris(ortho-substituted aryl) phosphite mole concentration of the reaction mixture is preferably adjusted to 0.9 to 15 times the sum of the peroxide mole concentration of the reaction mixture and the rhodium atom mole concentration of the reaction mixture, the peroxide being originally contained in the raw material. The phosphite mole concentration is more preferably 1 to 10 times, still more preferably 1 to 5 times from the viewpoints of rate of reaction and stability of the catalyst. The peroxide mole concentration of the reaction mixture, the peroxide being originally contained in 2,5-dihydrofuran, may be calculated through the method described in the Examples hereinbelow.

The present invention is carried out in the presence of hydrogen. As described above, the hydrogen pressure is preferably 0.1 to 0.9 MPa (absolute pressure at ambient temperature). From the viewpoint of cost, the hydrogen pressure is more preferably 0.11 to 0.6 MPa (absolute pressure at ambient temperature), still more preferably 0.11 to 0.4 MPa (absolute pressure at ambient temperature). In addition to hydrogen a gas inert to the reaction such as argon or nitrogen may also be used. When such an inert gas is used with hydrogen, the aforementioned absolute pressure means the partial pressure of hydrogen.

As described above, the reaction temperature is preferably 60 to 180°C, more preferably 90 to 160°C, still more preferably 90 to 130°C. When the temperature conditions are satisfied, the catalytic activity can be maintained at high level, leading to high rate of reaction, which is preferred. Needless to say, since reaction is performed at the aforementioned temperature after the aforementioned hydrogen pressure has been attained, the total pressure inside the reactor is elevated after elevation of temperature.

In the isomerization reaction of the present invention, by-products other than the target compound may be produced under certain reaction conditions. For example, when the target compound is 2,3-dihydrofuran, compounds such as 2-hydroxytetrahydrofuran, butyrolactone (i.e., dehydro-form of 2-hydroxytetrahydrofuran), and 4-hydroxybutanoic acid (i.e., hydrate of butyrolactone) may be by-produced. In order to adverse effects of such carboxylic acids, a tertiary amine such as trioctylamine may be added to the reaction mixture. Among tertiary amines, a tertiary amine that remains as a distillation residue in the removal of low-boiling-point compounds including reaction products to recover the rhodium compound and the monodentate tris(ortho-substituted aryl) phosphite; e.g., trioctylamine having a boiling point of 100°C or higher at normal pressure, is preferred.

When a tertiary amine is employed, generally, the amount thereof is preferably 1 to 2 times by mole with respect to the acid concentration of the reaction mixture, more preferably 1 to 1.5 times by mole. The acid mole concentration of the reaction mixture may be calculated through the method described in the Examples hereinbelow.

As described above, since the rhodium compound employed in the present invention is an expensive transition noble metal catalyst, the compound is preferably used repeatedly in the reaction.

In the case where the target vinyl ether compound is 2,3-dihydrofuran, in one mode of repeated use of the rhodium catalyst, substances contained in the reaction mixture after completion of reaction; e.g., 2,3-dihydrofuran, tetrahydrofuran (by-product), 2-hydrcoxytetrahydrofuran, 2,5-dihydrofuran (unreacted raw material), furan (originally contained in raw material), and water (originally contained in raw material), are distilled out through simple distillation, and the residue containing a rhodium compound and monodentate tris(ortho-substituted aryl) phosphite is recovered. Then, this residue containing the catalysts is added to the raw material, and the mixture is allowed to react.

From the viewpoint of production cost, the aforementioned rhodium compound and monodentate tris(ortho-substituted aryl) phosphite are preferably used repeatedly four times or more.

The present invention may be carried out in the absence of solvent, or may be carried out in the presence of a solvent that is inert in the reaction system. Examples of the inert solvent include saturated aliphatic hydrocarbons such as octane, nonane, and decane; aromatic hydrocarbons such as benzene, toluene, and xylene; and ethers such as tetraethylene glycol dimethyl ether and dioxane. These solvents may be used singly or in combination of two or more species.

No particular limitation is imposed on the reaction format of the present invention, and a batch manner or a continuous manner may be employed.

In one preferred mode of the batch manner, an allyl ether compound, a rhodium compound, a monodentate tris(ortho-substituted aryl) phosphite, an optional solvent, and an optional tertiary amine are mixed together in the presence of hydrogen (preferably under the aforementioned hydrogen pressure), and heating the mixture to the aforementioned temperature.

After completion of reaction, the reaction mixture is subjected to simple distillation, to hereby separate the

catalyst, and the distillate is rectified, whereby a high-purity vinyl ether compound of interest can be yielded.
The thus-obtained vinyl ether compound, for example, 2,3-dihydrofuran or 1,7-octadienyl methyl ether, is useful as a raw material, an intermediate, etc. for producing pharmaceuticals, agrochemicals, polymers, etc.

Among allyl ether compounds serving as a raw material of the present invention, 2,5-dihydrofuran used in the invention may be in the form of a distillation residue containing 2,5-dihydrofuran, which is produced through a method in which cis-2-butene-1,4-diol is dehydrated and subjected to simple distillation (i.e., a method disclosed in Japanese Patent Application Laid-Open (*kokai*) No. Hei 9110850), and the residue may be used without any further treatment.
In this case, the raw material contains impurities such as crotonaldehyde, furan, tetrahydrofuran, and water. In order to remove these impurities having small difference in boiling point from that of 2,5-dihydrofuran, a high-performance distillation tower is required, imposing high cost on the facility, and utilities unavoidably increase. When purification only through simple distillation is performed, these impurities cannot be removed. However, since the impurities do not affect the isomerization reaction, the distillation residue recovered through the aforementioned procedure is preferably used in the present invention from the viewpoint of simplicity.
Generally, the aforementioned distillation residue has an impurity content of about 0.1 to about 7 mass% (furan), about 0.5 to about 23 mass% (water), and about 0.01 to about 5 mass% (crotonaldehyde). Hereinafter, 2,5-dihydrofuran containing such impurities may be referred to as "crude 2,5-dihydrofuran."
Among allyl ether compounds serving as a raw material of the present invention, 2,7-octadienyl methyl ether may be produced through a method disclosed in Japanese Patent Application Laid-Open (*kokai*) No. 2005-95850.

## Examples

The present invention will next be described in more detail by way of examples, which should not be construed as limiting the invention thereto. Unless otherwise specified, in the following Examples, pressure denotes an absolute pressure value.
Also in the following Examples, each ingredient concentration (other than water content) of the reaction mixture after completion of reaction was determined through gas chromatography, and the water content was determined through the Karl Fischer's method.
The employed gas chromatographic analysis conditions, the Karl Fischer's water content measurement method, and the peroxide value measurement method will be described in detail.

### [Gas chromatographic analysis]

Apparatus: GC-9A (Shimadzu Corporation)
Column used: CBP-1 (50 m) (Shimadzu Corporation)
Analysis conditions: injection temp. of 50°C, detection temp. of 250°C
Temperature elevation profile: 50°C (10 minutes), elevation to 250°C at 15°C/min, 250°C (10 minutes)

### [Karl Fischer's water content measurement]

Apparatus: Hiranuma Microwater content measurement apparatus AQ-6 (Hiranuma Sangyo Corporation)
Causing liquid: Hydranal Aqualite RO (Sigma Aldrich Japan)
Counter liquid: Aqualite CN (canto Chemical Co., Inc.)
Sample amount: 1 g precisely weighed

### [Determination of peroxide value]

Peroxide value was determined through the following modified method of [Determination method A] (2), disclosed in "Shin-Jikken Kagaku Kouza 15, Oxidation and Reduction [1-2]," p. 685, line 12 (edited by The Chemical Society of Japan).
Specifically, KI-saturated 2-propanol (10 mL) and crude 2,5-DHF (1 to 4 g) were dissolved in 2-propanol containing 10 mass% acetic acid (25 to 30 mL), and the mixture was refluxed for five minutes or longer. The refluxed product was cooled to room temperature, and water (5 mL) was added thereto. The product was titrated with 0.01N aqueous sodium thiosulfate.

$$\text{Peroxide value [meq/kg]} = 10 \times A \times F / W$$

(wherein A represents a 0.01N sodium thiosulfate titration value, F represents a factor of the 0.01N sodium thiosulfate, and W represents a mass of the sample)
The thus-obtained peroxide value was divided by specific weight, whereby the peroxide mole concentration (mmol/L) of the reaction mixture, the peroxide being originally contained in the raw material, was obtained.

The percent conversion of an allyl ether compound and the percent selectivity to each ingredient contained in the reaction mixture after completion of reaction were calculated as follows.

[Calculation of percent conversion and percent selectivity]

Percent conversion and percent selectivity were calculated from area percentages of ingredients (excluding solvent) obtained through gas chromatographic analysis. Specifically, the following equations were employed to calculate percent conversion and percent selectivity.

$$\text{Percent conversion} = [(\text{area\% of allyl ether compound at feeding} - \text{area\% of allyl ether compound at sampling})/\text{area\% of allyl ether compound at feeding}] \times 100$$

$$\text{Percent selectivity} = [\text{area\% of each compound at sampling}/(\text{area\% of allyl ether compound at feeding} - \text{area\% of allyl ether compound at sampling})] \times 100$$

The term "area%" refers to the ratio (%) of a peak area attributed to a specific ingredient calculated by means of an integrator to the total peak area attributed to all ingredients or a peak area attributed to an employed ingredient.

[Determination of acid concentration]

A reaction mixture (about 10 g) was precisely weighed, and titrated with a 0.01-mol/L aqueous sodium hydroxide solution by means of an automatic titrator "AUT-501" (TOA Corporation). Through potentiometric titration, the inflection point within a pH range of 7 to 11 was determined.

<Production Example 1> Production of crude 2,5-dihydrofuran (1)

A Teflon (registered trademark) bag containing therein E30N4 [γ-alumina (ring-form pellets having inner diameter 2 mm, outer diameter: 5 mm, and height: 3.8 mm), product of JGC Corporation] (34 g) was placed in and fixed at a three-neck flask (capacity: 1 L) equipped with an electromagnetic stirrer and a distillation outlet. A distillation column [Helipack No. 2 (To-Toku Engineering Corporation), inner diameter: 25 mm, length: 300 mm, and theoretical plate: 20] was connected to the flash, and a water-cooling reflux condenser was connected to the distillation column.
To the aforementioned flask, cis-2-butene-1,4-diol (500 mL, 540 g, 6.1 mol) was fed, and heated to 195°C under nitrogen at ambient pressure with stirring. Through refluxing, dihydrofuran and low-boiling-point by-products (e.g., tetrahydrofuran, water, and crotonaldehyde) were distilled out while the distillation temperature was maintained at 83 to 86°C (ambient pressure). Also, cis-2-butene-1,4-diol was continuously fed to the flask so that the volume of the reaction mixture in the flask was maintained at 500 mL. The cis-2-butene-1,4-diol concentration of the reaction mixture immediately after start of reaction was found to be 99 mass%. The reaction was performed for eight hours, to thereby yield 753 mL of crude 2,5-dihydrofuran (hereinafter referred to as "crude 2,5-dihydrofuran (1)").
Through analysis, the crude 2,5-dihydrofuran (1) was found to have a purity of 93.3 mass% and contain furan (1.8

mass%), tetrahydrofuran (0.4 mass%), crotonaldehyde (0.5 mass%), and water (4.0 mass%), with a peroxide concentration of ≤0.1 mmol/L (peroxide value of ≤0.1 meq/kg).

<Production Example 2> Production of crude 2,5-dihydrofuran (2)

The reaction of Production Example 1 was repeated, except that the reaction temperature was changed to 203°C. Through refluxing, 2,5-dihydrofuran and low-boiling-point by-products (e.g., tetrahydrofuran, water, and crotonaldehyde) were distilled out while the distillation temperature was maintained at 93 to 96°C (ambient pressure). The reaction was performed for five hours, to thereby yield 835 mL of crude 2,5-dihydrofuran (hereinafter referred to as "crude 2,5-dihydrofuran (2)").
Through analysis, the crude 2,5-dihydrofuran (2) was found to have a purity of 85.1 mass% and contain furan (2.2 mass%), tetrahydrofuran (1.0 mass%), crotonaldehyde (1.1 mass%), and water (9.5 mass%), with a peroxide concentration of 1.14 mmol/L (peroxide value of 1.2 meq/kg).

<Production Example 3> Production of crude 2,5-dihydrofuran (3) (scale-up)

A heat-exchanger (diameter: 600 mm) in which 40 SUS 304 pipes (diameter: 25.4 mm, length: 2,000 mm) were juxtaposed and welded was filled with E30N4 [γ-alumina (ring-form pellets having inner diameter 2 mm, outer diameter: 5 mm, and height: 3.8 mm), product of JGC Corporation] (17 kg). The heat-exchanger was connected via piping to a distillation tower (Techno-Pack A4, product of Mitsui & Co., Ltd., height: 18.87 m, tower diameter: 300 mm, filled column with theoretical plate: 20) and to a circulation pump (maximum output: 30 $m^3$/hour, Teikoku Electric Mfg. Co., Ltd.). An additional heat-exchanger for condensation (heat conduction area: 5 $m^2$) was connected to the top of the distillation tower. To the aforementioned heat-exchanger and the bottom layer of the distillation tower, cis-2-butene-1,4-diol (200 L, 240 kg, 2,727 mol) was fed, and heated to 195°C under nitrogen at ambient pressure, while the compound was circulated between the heat-exchanger and the bottom layer of the distillation tower by means of the circulation pump at 15 $m^3$/hour. Through refluxing, 2,5-dihydrofuran and low-boiling-point by-products (e.g., tetrahydrofuran, water, and crotonaldehyde) were distilled out while the distillation temperature was maintained at 83 to 86°C (ambient pressure). Also, cis-2-butene-1,4-diol was continuously fed to the flask so that the volume of the reaction mixture in the flask was maintained at 200 L. The cis-2-butene-1,4-diol concentration of the reaction mixture immediately after start of reaction was found to be 99 mass%. The reaction was performed for 28 hours, to thereby yield 1,142 kg of crude 2,5-dihydrofuran (hereinafter referred to as "crude 2,5-dihydrofuran (3").
Through analysis, the crude 2,5-dihydrofuran (3) was found to have a purity of 85.1 mass% and contain furan (2.2 mass%), tetrahydrofuran (1.0 mass%), crotonaldehyde (1.1 mass%), and water (9.5 mass%), with a peroxide concentration of 1.14 mmol/L (peroxide value of 1.2 meq/kg, equivalent to 2.3 mol of 2,5-dihydrofuran).

<Example 1>

An autoclave (capacity; 300 mL) equipped with an electromagnetic stirrer, a gas inlet, and an opening for sampling was used. Into the autoclave, Rh(acac) $(CO)_2$ (20.6 mg, 0.08 mmol), tris(2,4-di-t-butylphenyl) phosphite (125.2 mg, 0.2 mmol; i.e., 2 to 2.5 times the sum of the peroxide mole concentration of the reaction mixture (≤0.02 mmol/L) and the rhodium atom mole concentration of the reaction mixture (0.08 mmol/L), the peroxide being originally contained in the raw material), and 2,5-dihydrofuran (1), produced in Production Example 1, (200 mL), were fed in a nitrogen atmosphere under air-tight conditions. The atmosphere of the autoclave was changed to hydrogen at a hydrogen pressure (absolute pressure) of 0.25 MPa. When the temperature inside the autoclave was elevated to 110°C, the total pressure was elevated to 0.55 MPa. While hydrogen was fed to the autoclave so as to maintain this total pressure, reaction was performed for four hours. The obtained reaction mixture was analyzed through gas chromatography. Table 1 shows the results.

<Example 2>

The reaction and analysis of Example 1 were repeated, except that the hydrogen pressure (absolute pressure) was adjusted to 0.4 MPa (a total pressure of 0.7 MPa at 110°C). Table 1 shows the results.

<Example 3>

The reaction and analysis of Example 1 were repeated, except that the hydrogen pressure (absolute pressure) was adjusted to 0.6 MPa (a total pressure of 0.9 MPa at 110°C). Table 1 shows the results.

<Example 4>

The reaction and analysis of Example 1 were repeated, except that the hydrogen pressure (absolute pressure) was adjusted to 0.11 MPa (a total pressure of 0.4 MPa at 110°C). Table 1 shows the results.

<Example 5>

The reaction and analysis of Example 4 were repeated, except that the reaction time was changed to 16 hours. Table 1 shows the results.

<Comparative Example 1>

The reaction and analysis of Example 1 were repeated, except that "substitution of the atmosphere of the autoclave with hydrogen at a hydrogen pressure (absolute pressure) of 0.25 MPa" was changed to "adjustment of the nitrogen pressure (absolute pressure) inside the autoclave to 0.25 MPa (a total pressure of 0.55 MPa at 110°C). Table 1 shows the results.

<Example 6>

The reaction and analysis of Example 1 were repeated, except that the hydrogen pressure (absolute pressure) was changed to 1.0 MPa (a total pressure of 1.3 MPa at 110°C). Table 1 shows the results.

<Example 7>

The reaction and analysis of Example 1 were repeated, except that "substitution of the atmosphere of the autoclave with hydrogen at a hydrogen pressure (absolute pressure) of 0.25 MPa" was changed to "substitution of the atmosphere of the autoclave with hydrogen and carbon monoxide at a hydrogen pressure (absolute pressure) of 0.2 MPa and a carbon monoxide pressure (absolute pressure) of 0.2 MPa (a total pressure of 0.7 MPa at 110°C)." Table 1 shows the results.

<Example 8>

The reaction and analysis of Example 1 were repeated, except that "substitution of the atmosphere of the autoclave with hydrogen at a hydrogen pressure (absolute pressure) of 0.25 MPa" was changed to "substitution of the atmosphere of the autoclave with hydrogen at a hydrogen pressure (absolute pressure) of 0.25 MPa" was changed to "substitution of the atmosphere of the autoclave with hydrogen and carbon monoxide at a hydrogen pressure (absolute pressure) of 0.23 MPa and a carbon monoxide pressure (absolute pressure) of 0.08 MPa (a total pressure of 0.6 MPa at 110°C)." Table 1 shows the results.

[Table 1]

Table 1

| | Reaction time (h) | Hydrogen pressure (MPa) | Othergas pressure (MPa) | Conversion (%) 2,5DHF | Selectivity (%) | | | | |
|---|---|---|---|---|---|---|---|---|---|
| | | | | | 2,3DHF | F | THF | 2HTHF | 3FTHF + 2FTHF |
| Ex. 1 | 4 | 0.25 | - | 94.0 | 92.2 | 0 | 2.5 | 5.3 | 0 |
| Ex. 2 | 4 | 0.4 | - | 97.0 | 86.0 | 0 | 4.5 | 9.5 | 0 |
| Ex. 3 | 4 | 0.6 | - | 98.5 | 82.0 | 0 | 8.7 | 9.3 | 0 |
| Ex. 4 | 4 | 0.11 | - | 25.2 | 97.9 | 0.8 | 0.1 | 1.2 | 0 |
| Ex. 5 | 16 | 0.11 | - | 90.5 | 82.7 | 3.0 | 0.1 | 14.3 | 0 |
| Comp. Ex. 1 | 4 | 0 | $N_2$ 0.25 | 3.2 | 75.2 | 2.0 | 0 | 22.8 | 0 |
| Ex. 6 | 4 | 1.0 | - | 99.8 | 52,4 | 0 | 45.3 | 2.3 | 0 |
| Ex. 7 | 4 | 0.2 | CO 0.2 | 96.5 | 82.2 | 0 | 2.2 | 11.2 | 4.4 |

(continued)

| | Reaction time (h) | Hydrogen pressure (MPa) | Othergas pressure (MPa) | Conversion (%) 2,5DHF | Selectivity (%) | | | | |
|---|---|---|---|---|---|---|---|---|---|
| | | | | | 2,3DHF | F | THF | 2HTHF | 3FTHF + 2FTHF |
| Ex. 8 | 4 | 0.23 | CO 0.08 | 96.7 | 85.4 | 0 | 4.3 | 9.5 | 0.8 |

[Note]

2, SDHF: 2,5-dihydrofuran
2,3DHF: 2,3-dihydrofuran
F: furan
THF: tetrahydrofuran
2HTHF: 2-hydroxytetrahydrofuran
3FTHF: 3-formyltetrahydrofuran
2FTHF: 2-formyltetrahydrofuran

As is clear from Table 1, when the hydrogen pressure (absolute pressure) at ambient temperature was 0.1 MPa or higher, percent conversion of 2,5-dihydrofuran was high, and percent selectivity to 2,3-dihydrofuran was high (Examples 1 to 8). Particularly when the hydrogen pressure (absolute pressure) at ambient temperature was adjusted to 0.1 to 0.9 MPa (Examples 1 to 5), percent selectivity to 2,3-dihydrofuran was considerably high. In Example 4, in which the hydrogen pressure (absolute pressure) was 0.11 MPa, percent conversion to 2,5-dihydrofuran was reduced. However, as is the case of Example 5, a high percent conversion was attained through prolongation of the reaction time. When the hydrogen pressure (absolute pressure) was adjusted to 0.1 to 0.9 MPa by use of hydrogen and carbon monoxide (Examples 7 and 8), percent conversion of 2,5-dihydrofuran and percent selectivity to 2,3-dihydrofuran were very high.
In contrast, when the hydrogen pressure (absolute pressure) at ambient temperature was lower than 0.1 MPa (Comparative Example 1), percent conversion of 2,5-dihydrofuran was considerably reduced, and percent selectivity to 2,3-dihydrofuran was reduced.

<Example 9>

The reaction and analysis of Example 1 were repeated, except that tris(3-methyl-6-t-butylphenyl) phosphite (3M6BP) (107.8 mg, 0.2 mmol) was used instead of tris(2,4-di-t-butylphenyl) phosphite (125.2 mg, 0.2 mmol). Table 2 shows the results.

<Example 10>

The reaction and analysis of Example 1 were repeated, except that tris(2-t-butylphenyl) phosphite (2TBP) (95.7 mg, 0.2 mmol) was used instead of tris(2,4-di-t-butylphenyl) phosphite (125.2 mg, 0.2 mmol). Table 2 shows the results.

<Comparative Example 2>

The reaction and analysis of Example 1 were repeated, except that triphenylphosphine (52.4 mg, 0.2 mmol) was used instead of tris(2,4-di-t-butylphenyl) phosphite (125.2 mg, 0.2 mmol). Table 2 shows the results.

<Comparative Example 3>

The reaction and analysis of Example 1 were repeated, except that triphenyl phosphite (62 mg, 0.2 mmol) was used instead of tris(2,4-di-t-butylphenyl) phosphite (125.2 mg, 0.2 mmol) Table 2 shows the results.

<Comparative Example 4>

The reaction and analysis of Example 1 were repeated, except that tris(4-methylphenyl) phosphite (70.5 mg, 0.2 mmol) was used instead of tris(2,4-di-t-butylphenyl) phosphite (125.2 mg, 0.2 mmol). Table 2 shows the results.

[Table 2]

Table 2

| | P ligand | Reaction time (h) | Conversion (%) 2,5DHF | Selecticity (%) | | | | |
|---|---|---|---|---|---|---|---|---|
| | | | | 2,3DHF | F | THF | 2HTHF | Others |
| Ex. 9 | 3M6BP | 4 | 94.8 | 94.2 | ≤0.1 | 2.3 | 3.3 | 0.2 |
| Ex. 10 | 2TBP | 4 | 95.8 | 93.9 | ≤0.1 | 3.3 | 2.8 | 0 |
| Comp. Ex. 2 | triphenylphosphine | 4 | 66.1 | 78.5 | ≤0.1 | 16.5 | 4.5 | 0.5 |
| Comp. Ex. 3 | triphenyl phosphite | 4 | 58.4 | 84.3 | ≤0.1 | 4.5 | 10.7 | 0.5 |
| Comp. Ex. 4 | tris(4-methylphenyl) phosphite | 4 | 64.3 | 79.5 | ≤0.1 | 15,5 | 4.7 | 0.3 |

[Note]

3M6BP: tris(3-methyl-6-t-butylphenyl) phosphite
2TBP: tris(2-t-butylphenyl) phosphite
2,5DHF: 2,5-dihydrofuran
2,3DHF: 2,3-dihydrofuran
F: furan
THF: tetrahydrofuran
2HTHF: 2-hydroxytetrahydrofuran

As is clear from Table 2, in Examples 9 and 10, which employed a monodentate tris(ortho-substituted aryl) phosphite, percent conversion of dihydrofuran and percent selectivity to 2,3-dihydrofuran were high. In contrast, in Comparative Examples 2 to 4, which employed another phosphorus ligand having no substituent at the ortho-position of the aryl group of phosphite, both percent conversion of 2,5-dihydrofuran and percent selectivity to 2,3-dihydrofuran were reduced.

<Example 11>

The reaction and analysis of Example 1 were repeated, except that tris(2,4-di-t-butylphenyl) phosphite (0.08 mmol, 1 time the sum of the peroxide (originally contained in the raw material) mole concentration of the reaction mixture and the rhodium atom mole concentration) was used instead of tris(2,4-di-t-butylphenyl) phosphite (0.2 mmol, 2.5 times the sum of the peroxide mole concentration of the reaction mixture and the rhodium atom mole concentration). Table 3 shows the results.

<Example 12>

The reaction and analysis of Example 11 were repeated, except that the reaction time was changed to six hours. Table 3 shows the results.

<Example 13>

The reaction and analysis of Example 1 were repeated, except that tris(2,4-di-t-butylphenyl) phosphite (0.8 mmol, 10 times the sum of the peroxide (originally contained in the raw material) mole concentration of the reaction mixture and the rhodium atom mole concentration) was used instead of tris(2,4-di-t-butylphenyl) phosphite (0.2 mmol). Table 3 shows the results.

<Example 14>

The reaction and analysis of Example 13 were repeated, except that the reaction time was changed to six

hours. Table 3 shows the results.

<Example 15>

The reaction and analysis of Example 13 were repeated, except that crude 2,5-dihydrofuran (1) (200 mL) was changed to crude 2,5-dihydrofuran (2), produced in Production Example 2, (200 mL). Table 3 shows the results.

<Example 16>

The reaction and analysis of Example 1, were repeated, except that tris(2,4-di-t-butylphenyl) phosphite (2 mmol), 25 times the sum of the peroxide mole (originally contained in the raw material) concentration of the reaction mixture and the rhodium atom mole concentration) was used instead of tris(2,4-di-t-butylphenyl) phosphite (0.2 mmol). Table 3 shows the results.

<Example 17>

The reaction and analysis of Example 16 were repeated, except that the reaction time was changed to six hours. Table 3 shows the results.

<Example 18>

The reaction and analysis of Example 15 were repeated, except that tris(3-methyl-6-t-butylphenyl) phosphite (0.02 mmol, 0.25 times the sum of the peroxide (originally contained in the raw material) mole concentration of the reaction mixture and the rhodium atom mole concentration) was used instead of tris(2,4-di-t-butylphenyl) phosphite (0.8 mmol). Table 3 shows the results.

<Comparative Example 5>

The reaction of Example 1 was repeated, except that no tris(2,4-di-t-butylphenyl) phosphite was added. In Comparative Example 5, no reaction occurred. (see Table 3).

[Table 3]

Table 3

| | Reaction time (h) | Tris(substituted aryl) phosphite mole concentration (mmol/L) | Peroxide concentration (mmol/L) | Mole concentration ratio* | Conversion (%) | Selectity (%) | | | |
|---|---|---|---|---|---|---|---|---|---|
| | | | | | 2,5DHF | 2,3DHF | THF | 2HTHF | others |
| Ex. 11 | 4 | 0.4 | <0.2 | 1 | 79.5 | 93.3 | 2.7 | 4.0 | 0 |
| Ex. 12 | 6 | 0.4 | <0.2 | 1 | 94.5 | 92.1 | 3.3 | 4.4 | 0.2 |
| Ex. 13 | 4 | 4 | <0.2 | 10 | 64.8 | 93.5 | 3.0 | 3.5 | 0 |
| Ex. 14 | 6 | 4 | <0.2 | 10 | 88.2 | 92.7 | 3,1 | 4.2 | 0 |
| Ex. 15 | 4 | 4 | 1.2 | 2.5 | 91.5 | 94.6 | 0.4 | 5.0 | 0 |
| Ex. 16 | 4 | 10 | <0.2 | 25 | 12.5 | 94.7 | 2.3 | 3.0 | 0 |
| Ex. 17 | 6 | 10 | <0.2 | 25 | 19.0 | 94.5 | 2.3 | 3.2 | 0 |
| Ex. 18 | 4 | 0.1 | 1.2 | 0.25 | 21.2 | 70.0 | 15.5 | 14.5 | 0 |
| Comp. Ex. 5 | 4 | 0 | <0.2 | 0 | 0 | - | - | - | - |

*Tris(substituted aryl) phosphite mole concentration to the sum of peroxide mole concentration and rhodium atom mole concentration

[Note]

2,5DHF: 2,5-dihydrofuran
2,3DHF: 2,3-dihydrofuran
THF: tetrahydrofuran
2HTHF: 2-hydroxytetrahydrofuran

As is clear from Table 3, when the monodentate tris(ortho-substituted aryl) phosphite mole concentration of the reaction mixture was 0.9 to 15 times the sum of the peroxide (originally contained in the raw material) mole concentration of the reaction mixture and the rhodium atom mole concentration (Examples 11 to 15), excellent rates of reaction and percent selectivity values were attained.
In Examples 16 to 18, where the monodentate tris(ortho-substituted aryl) phosphite mole concentration of the reaction mixture fell outside the range of 0.9 to 15 times the sum of the peroxide (originally contained in the raw material) mole concentration of the reaction mixture and the rhodium atom mole concentration, percent conversion of 2,5-dihydrofuran was reduced, but high percent selectivity to 2,3-dihydrofuran was attained.
In the case where no monodentate tris(ortho-substituted aryl) phosphite was added (Comparative Example 5), no reaction occurred.

<Example 19>

The reaction and analysis of Example 1 were repeated, except that trioctylamine (200 mg, 0.57 mmol) was further added. As a result, there were obtained a percent conversion 2,5-dihydrofuran of 85.3%, a percent selectivity to 2,3-dihydrofuran of 96.4%, a percent selectivity to tetrahydrofuran of 2.5%, and a percent selectivity to 2-hydroxytetrahydrofuran of 1.1%. The rate of reaction was slightly lower than that of Example 1, but percent selectivity to a by-product (2-hydroxytetrahydrofuran) was reduced, and percent selectivity to 2,3-dihydrofuran was enhanced.

<Example 20>

A jacketed reactor (capacity: 140 L, pressure capacity: 2.0 MPa) equipped with a gas inlet, an opening for sampling, a condenser, a distillate reservoir connected to the condenser, and a stirrer was employed. Into the reactor, $Rh(acac)(CO)_2$ (10.3 g, 41.8 mmol), tris(2,4-di-t-butylphenyl) phosphite (129 g, 206 mmol), crude 2,5-dihydrofuran (3), produced in Production Example 3, (30 L), and trioctylamine (80 g, 228 mmol) were fed in a nitrogen atmosphere under air-tight conditions. The atmosphere of the reactor was changed to hydrogen at a hydrogen pressure (absolute pressure) of 0.20 MPa. When the inside temperature of the reactor was elevated to 120°C, the total pressure was elevated to 0.5 MPa. Thereafter, crude 2,5-dihydrofuran (3) (70 L) was fed to the reactor at 36 L/hour over 1.7 hours by means of a feed pump. While hydrogen was fed to the reactor so as to maintain the inside pressure of the reactor, reaction was performed for 1.8 hours. The obtained reaction mixture was analyzed through gas chromatography. Table 4 shows the results.
After confirmation of completion of reaction through gas chromatographic analysis, water (25°C) was caused to pass through the jacket of the reactor, to thereby cool the reaction mixture to 30°C. The pressure of the reactor (0.2 MPa) was released via the condenser and a distillate reservoir, and then, the reaction mixture in the reactor was subjected to simple distillation to a volume of 10 L by heating the reactor to 70°C under nitrogen. Through distillation, low-boiling-point substances including a target compound were distilled out.
Subsequently, another aliquot of crude 2,5-dihydrofuran (3) (30 L) was fed the reactor. The atmosphere of the reactor was changed to hydrogen at a hydrogen pressure (absolute pressure) of 0.20 MPa, and the inside temperature of the reactor was elevated to 120°C. Crude 2,5-dihydrofuran (3) (60 L) was fed to the reactor at 36 L/hour over 1.7 hours by means of a feed pump. While hydrogen was fed to the reactor so as to maintain the inside pressure of the reactor, reaction was performed for 1.8 hours. The obtained reaction mixture was analyzed through gas chromatography. The sequence of the reaction, analysis, and distillation was repeated four times in total.
Each of the thus-obtained distillation residues was found to have a viscosity (at 30°C) of 0.1 Pa·s or less and encountered no difficulty in handling. Table 4 shows the results.
[Table 4]

Table 4

| Example 20 | Conversion (%) 2,5DHF | Selectivity (%) | | | |
|---|---|---|---|---|---|
| | | 2,3DHF | THF | 2HTHF | Others |
| 1st | 94.5 | 96.8 | 0.2 | 3.0 | 0 |
| 2nd | 99.8 | 93.2 | 0.4 | 3.2 | 3.2 |
| 3rd | 99.5 | 93.9 | 0.5 | 0 | 5.6 |
| 4th | 99.0 | 96.8 | 1.0 | 0 | 2.2 |

[Note]

2,5DHF: 2,5-dihydrofuran
2,3THF: 2,3-dihydrofuran
THF: tetrahydrofuran
2HTHF: 2-hydroxytetrahydrofuran

<Example 21>

An autoclave (capacity: 300 mL) equipped with an electromagnetic stirrer, a gas inlet, and an opening for sampling was employed. Into the autoclave, Rh(acac) $(CO)_2$ (20.6 mg, 0.08 mmol), tris(2,4-di-t-butylphenyl) phosphite (125.2 mg, 0.2 mmol), and crude 2,5-dihydrofuran (1), produced in Production Example 1, (200 mL) were fed in a nitrogen atmosphere under air-tight conditions. Subsequently hydrogen was fed to the autoclave so as to adjust the hydrogen pressure (absolute pressure) to 0.25 MPa (a total pressure of 0.55 MPa at 110°C). While hydrogen was fed to the autoclave so as to maintain the inside pressure of the reactor, reaction was performed for 4 hours. The obtained reaction mixture was analyzed through gas chromatography. Table 5 shows the results.
The thus-obtained reaction mixture was transferred to a three-neck flask (capacity: 300 mL) equipped with a Liebig condenser and heated to 70°C at normal pressured under nitrogen, whereby low-boiling-point substances including a compound of interest was distilled out. Through distillation, the reaction mixture was concentrated to a volume of 30 mL. The thus-recovered concentrated residue containing a catalyst was transferred again to the autoclave. By use of Rh (acac)$(CO)_2$ and tris(2,4-di-t-butylphenyl) phosphite, a set of the same reaction, analysis, and simple distillation was repeated four times in total.
After completion of the fourth reaction, the concentrated liquid was found to have a viscosity (at 30°C) of 0.35 Pa·s, making handling of the residue slightly difficult. The obtained reaction mixture was analyzed through gas chromatography. Table 5 shows the results.
[Table 5]

Table 5

| Example 21 | Conversion (%) 2,5DHF | Selectivity (%) | | | |
|---|---|---|---|---|---|
| | | 2,3DHF | THF | 2HTHF | Others |
| 1st | 96.7 | 92.6 | 2.5 | 4.8 | 0 |
| 2nd | 94.5 | 85.0 | 2.3 | 7.2 | 5.5 |
| 3rd | 90.3 | 83.5 | 2.0 | 7.2 | 7.3 |
| 4th | 88.5 | 81.4 | 2.0 | 7.1 | 9.5 |

[Note]

2,5DHF: 2,5-dihydrofuran
2,3DHF: 2,3-dihydrofuran

THF: tetrahydrofuran
2HTHF: 2-hydroxytetrahydrofuran

In Examples 19 and 20, in which tertiary amine (trioctylamine) was used, percent selectivity to 2-hydroxytetrahydrofuran and to other high-boiling-point compounds were small, and the catalyst can be repeatedly used, leading to reduction in production cost. In Example 21, in which no tertiary amine (trioctylamine) was used, percent selectivity to 2-hydroxytetrahydrofuran and to other high-boiling-point compounds tended to slightly increase due to repeated use of the catalyst, but high conversion and high selectivity were able to be attained.

<Comparative Example 6>

The reaction procedure was performed in accordance with the method disclosed in Patent Document 4. Specifically, triphenylphosphine (100 g, 382 mmol) was fed to an autoclave (capacity: 300 mL) equipped with an electromagnetic stirrer, a gas inlet, and an opening for sampling and melted at 120°C. Subsequently, crude 2,5-dihydrofuran (1), produced in production Example 1, (40 g, 319 mmol) and $HRh(PPh_3)_3(CO)$ (5 g, 5.4 mmol) were added to the autoclave, and hydrogen was fed to the autoclave so as to elevate the hydrogen pressure (absolute pressure) to 0.11 MPa. The autoclave was heated to 120°C, and then reaction was performed for 10 hours while hydrogen was fed in order to maintain the inside pressure of the autoclave. The thus-obtained reaction mixture was analyzed through gas chromatography. Table 6 shows the results.

[Table 6]

Table 6

| | P ligand | Reaction time (h) | Conversion (%) 2,5DHF | selectivity (%) | | | | |
|---|---|---|---|---|---|---|---|---|
| | | | | 2,3DHF | F | THF | 2HTHF | Others |
| Comp. Ex. 6 | triphenylphosphine | 4 | 53.2 | 88.2 | 0.8 | 1.0 | 9.0 | 1.0 |
| | | 10 | 91.2 | 65.6 | 5.4 | 6.8 | 22.2 | 0 |

[Note]

2,5DHF: 2,5-dihydrofuran
2,3DHF: 2,3-dihydrofuran
F: furan
THF: tetrahydrofuran
2HTHF: 2-hydroxytetrahydrofuran

As is clear from Table 6, when the conditions disclosed in Patent Document 4 were employed, percent selectivity was lower, as compared with Example 4 of the present invention in which the same hydrogen pressure was employed. In Comparative Example 6, when the percent conversion was designed to increase, percent selectivity further decreased. In addition, although a rhodium catalyst was used in an amount 67.5 times the amount employed in Example 4, the rate of reaction was unsatisfactory.

<Example 22>

An autoclave (capacity: 300 mL) equipped with an electromagnetic stirrer, a gas inlet, and an opening for sampling was used. Into the autoclave, $Rh(acac)(CO)_2$ (220.6 mg, 0.08 mmol) and tris(2,4-di-t-butylphenyl) phosphite (500.8 mg, 0.8 mmol; i.e., 10 times the sum of the peroxide mole concentration of the reaction mixture (≤0.02 mmol/L) and the rhodium atom mole concentration of the reaction mixture (0.4 mmol/L), the peroxide being originally contained in the raw material), were fed and dissolved in toluene (100 mL). Then, 2,7-octadienyl methyl ether (100 mL) was fed to the autoclave. The above procedure was performed in a nitrogen atmosphere under air-tight conditions.
The atmosphere of the autoclave was changed to hydrogen at a hydrogen pressure (absolute pressure) of 0.4 MPa. When the temperature inside the autoclave was elevated to 120°C, the total pressure was elevated to 0.5 MPa. While hydrogen was fed to the autoclave so as to maintain this total pressure, reaction was performed for one hour. The obtained reaction mixture was analyzed through gas chromatography. Table 7 shows the results.

[Table 7]

Table 7

| | P ligand | Reaction time (h) | Conversion (%) 2,7MODE | Selectivity (%) | | | | |
|---|---|---|---|---|---|---|---|---|
| | | | | 1,7-MODE | 7-OEL | Other unsatd. ethers | Satd. ethers | Others |
| Ex. 22 | Tris(2,4-di-t-butylphenyl) phosphite | 1 | 97 | 64 | 1 | 1 | 9 | 25 |

[Note]

2.7MODE: 2,7-octadienyl methyl ether
1,7MODE: 1,7-octadienyl methyl ether
7-OEL: 7-octenal
Other unsaturated ethers: 7-octenyl methyl ether, 2,6-octadienyl methyl ether, etc.
Saturated ethers: n-octyl methyl ether

As is clear from Table 7, when 2,7-octadienyl methyl ether was used as the allyl ether compound serving as a raw material, high percent conversion and high percent selectivity were attained by use of a rhodium compound in a small amount.

Industrial Applicability

The present invention enables to production, in an industrially advantageous manner, of vinyl ether compounds such as, for example, 2,3-dihydrofuran or 1,7-octadienyl methyl ether, which are useful as a raw material, an intermediate, etc. for producing pharmaceuticals, agrochemicals, polymers, etc.

## Claims

1. A method for producing a vinyl ether compound, **characterized in that** the method comprises isomerizing an allyl ether compound represented by formula (I) or (II):

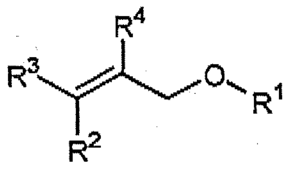

(I)

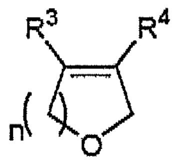

(II)

(wherein $R^1$ represents a C1 to C6 alkyl group; each of $R^2$, $R^3$, and $R^4$ independently represents a hydrogen atom, a C1 to C6 alkyl group, or a C3 to C6 alkenyl group; and n is 1 or 2) in the presence of hydrogen, a monodentate tris(ortho-substituted aryl) phosphite, and a rhodium compound.

2. A method for producing a vinyl ether compound according to claim 1, wherein the monodentate tris(ortho-substituted aryl) phosphite mole concentration of the reaction mixture is adjusted to 0.9 to 15 times the sum of the peroxide mole concentration of the reaction mixture and the rhodium atom mole concentration of the reaction mixture, the peroxide being originally contained in the raw material, and isomerization is performed at a reaction temperature of

60 to 180°C in the absence of carbon monoxide after adjusting the hydrogen pressure to 0.1 to 0.9 MPa (absolute pressure).

**3.** A method for producing a vinyl ether compound according to claim 1 or 2, wherein the reaction mixture has a rhodium atom mole concentration of 0.01 to 2 mmol/L.

**4.** A method for producing a vinyl ether compound according to claim 1 or 2, wherein isomerization is performed further in the presence of a tertiary amine having a boiling point of 100°C or higher.

**5.** A method for producing a vinyl ether compound according to claim 1 or 2, wherein the monodentate tris(ortho-substituted aryl) phosphite is at least one species selected from among tris(2-t-butylphenyl) phosphite, tris(3-methyl-6-t-butylphenyl) phosphite, and tris(2,4-di-t-butylphenyl) phosphite.

**6.** A method for producing a vinyl ether compound according to claim 1 or 2, wherein the allyl ether compound serving as a raw material is 2,5-dihydrofuran containing, as impurities, furan in an amount of 0.1 to 7 mass%, water in an amount of 0.5 to 23 mass%, and crotonaldehyde in an amount of 0.01 to 5 mass%.

**INTERNATIONAL SEARCH REPORT**

International application No.
PCT/JP2009/058570

A. CLASSIFICATION OF SUBJECT MATTER
*C07D307/28*(2006.01)i, *C07B61/00*(2006.01)i, *C07C41/32*(2006.01)i, *C07C43/15* (2006.01)i

According to International Patent Classification (IPC) or to both national classification and IPC

B. FIELDS SEARCHED

Minimum documentation searched (classification system followed by classification symbols)
C07D307/28, C07B61/00, C07C41/32, C07C43/15

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2009 |
| Kokai Jitsuyo Shinan Koho | 1971-2009 | Toroku Jitsuyo Shinan Koho | 1994-2009 |

Electronic data base consulted during the international search (name of data base and, where practicable, search terms used)
CA/REGISTRY(STN), CAplus(STN), CASREACT(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| A | JP 9-508109 A (BASF AG.), 19 August, 1997 (19.08.97), The entire specification & US 5705707 A & EP 739332 A & WO 1995/019334 A1 | 1-6 |
| A | US 5254701 A (Eastman Kodak Co.), 19 October, 1993 (19.10.93), The entire specification & EP 586522 A1 & WO 1992/020667 A1 | 1-6 |
| A | LEUNG, Dennis H., Highly Selective Supramolecular Catalyzed Allylic Alcohol Isomerization, J. AM. CHEM. SOC., 2007, Vol.129, No.10, p.2746-2747 | 1-6 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 16 July, 2009 (16.07.09) | 28 July, 2009 (28.07.09) |
| Name and mailing address of the ISA/ Japanese Patent Office | Authorized officer |
| Facsimile No. | Telephone No. |

Form PCT/ISA/210 (second sheet) (April 2007)

**INTERNATIONAL SEARCH REPORT**

International application No.
PCT/JP2009/058570

C (Continuation). DOCUMENTS CONSIDERED TO BE RELEVANT

| Category* | Citation of document, with indication, where appropriate, of the relevant passages | Relevant to claim No. |
|---|---|---|
| A | GILBERTSON, Scott R., Rhodium Catalyzed Intramolecular [4+2] Cycloisomerization Reactions, Tetrahedron Letters, 1998, Vol.39, p.2075-2078 | 1-6 |
| A | YAMAMOTO, Yasunori, STEREOSELECTIVE ISOMERIZATION OF UNSYMMETRICAL DIALLYL ETHERS TO ALLYL (E)-VINYL ETHERS BY A CATIONIC IRIDIUM CATALYST, SYNTHETIC COMMUNICATIONS, 2000, Vol.30, No.13, p.2383-2391 | 1-6 |
| A | GILBERTSON, Scott R., Rhodium-Catalyzed Asymmetric [4+2] Cycloisomerization Reactions, J. Org. Chem., 1998, Vol.63, No.26, p.10077-10080 | 1-6 |
| A | JP 2000-355572 A (Mitsubishi Chemical Corp.), 26 December, 2000 (26.12.00), The entire specification & US 6300515 B1 | 1-6 |
| A | JP 10-505074 A (BASF AG.), 19 May, 1998 (19.05.98), The entire specification & US 5892125 A & EP 778819 A & WO 1996/007630 A1 | 1-6 |

Form PCT/ISA/210 (continuation of second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP HEI9157201 B
- JP 2000510118 W
- US 5254701 A
- JP HEI9508109 W
- JP HEI9110850 B
- JP 2005095850 A


### Non-patent literature cited in the description


- Shin-Jikken Kagaku Kouza 15, Oxidation and Reduction [1-2]. 685